# EUROPEAN PATENT APPLICATION

(11) **EP 1 795 191 A1**
(43) Date of publication of application: **13.06.2007**
(21) Application number: 05785791.4
(22) Date of filing: 21.09.2005
(51) Int. Cl.: A61K 31/401, A23K 1/16, A23L 1/305, A61P 17/16, A61P 43/00, C07D 207/16

(54) **ORAL PHARMACEUTICAL FOR DRY SKIN PREVENTION OR REMEDY**

(30) Priority: 21.09.2004 JP 2004273432
(71) Applicant: KYOWA HAKKO KOGYO CO., LTD., Chiyoda-ku, Tokyo 100-8185 (JP)
(72) Inventor: KAGAMI, Erika c/o Healthcare Products Development Center, Ibaraki 305-0841 (JP); MORISHITA, Koji c/o Healthcare Products Development Center, Ibaraki 305-0841 (JP); KAMIYA, Toshikazu, c/o Healthcare Products Development Center, Ibaraki 305-0841 (JP); KAMIMURA, Ayako c/o Healthcare Products Development Center, Ibaraki 305-0841 (JP)
(74) Representative: Vossius & Partner
(86) International application number: PCT/JP2005/017389
(87) International publication number: WO 2006/033355

(57) **Abstract**

The present invention can provide orally administered agents, foods and drinks, feeds, food additives or feed additives for preventing or treating a dry state of the skin, which comprise hydroxyproline, an N-acyl derivative of hydroxyproline, especially an N-acetyl derivative, an N-propionyl derivative, an N-butyryl derivative, an N-isobutyryl derivative, or a salt thereof as an active ingredient.

## Description

### Technical Field

The present invention relates to orally administered agents, foods and drinks, feeds, food additives and feed additives for preventing or improving a dry state of the skin, which comprise hydroxyproline, N-acyl derivatives of hydroxyproline or salts thereof as an active ingredient.

### Background Art

The skin is always exposed to various irritations from the external environment. The stratum corneum of the skin has barrier functions of preventing invasion of these irritations or foreign matters from the outside and preventing transpiration of water within the body. It has been known that water content in the stratum corneum of the skin of persons or animals in which such barrier functions are reduced is decreased. For example, it has been reported that the water content in the stratum corneum of the skin is decreased in patients with atopic dermatitis (refer to Non-patent Document 1) or with aging (refer to Non-patent Document 2).

As a method for maintaining or improving moistening property of the skin, a method in which the barrier functions of the stratum corneum are supplemented with a blocking agent such as a vaseline ointment or a water-in-oil emulsion, a method in which the water content of the stratum corneum is supplemented with a moistening agent such as sorbitol or glycerin, a method in which skin inflammation is suppressed with an antiinflammatory agent such as glycyrrhizic acid, a method in which skin cells are activated with, for example, vitamins or hormones, and the like have been so far used (refer to Non-patent Document 3).

It has been known that hydroxyproline, N-acyl derivatives of hydroxyproline or salts thereof in the external use exhibit an effect of suppressing the aging of the skin and an effect of improving skin condition (refer to Patent Document 1). It has been further known that the condition of the skin or the hair is improved by eating or drinking a mixed composition of L-proline, L-alanine and glycine which are major constitutive amino acids of collagen (refer to Patent Document 2). However, it has been unknown that the dry state of the skin is prevented or improved by oral ingestion of hydroxyproline, N-acyl derivatives of hydroxyproline or salts thereof.
Patent Document 1: WO00/51561
Patent Document 2: Japanese Published Unexamined Patent Application No. 2001-224334
Non-Patent Document 1: "Archives of Dermatology", 1991, vol. 127, p. 1689
Non-Patent Document 2: "Journal of Investigative Dermatology", 1990, vol. 95, p. 296
Non-Patent Document 3: "Fragrance Journal", 1999, vol. 10, p. 29

### Disclosure of the Invention

### Problems to be Solved by the Invention

An object of the invention is to provide safe orally administered agents, foods and drinks, feeds, food additives or feed additives for preventing or improving a dry state of the skin.

### Means for Solving the Problems

The present invention relates to the following (1) to (12).
(1) An orally administered agent for preventing or improving a dry state of the skin, which comprises hydroxyproline, an N-acyl derivative of hydroxyproline or a salt thereof as an active ingredient.
(2) The orally administered agent of the above (1), wherein the N-acyl derivative of hydroxyproline is an N-acetyl derivative, an N-propionyl derivative, an N-butyryl derivative or an N-isobutyryl derivative.
(3) A food and drink or a feed for preventing or improving a dry state of the skin, which comprises hydroxyproline, an N-acyl derivative of hydroxyproline or a salt thereof as an active ingredient.
(4) The food and drink or the feed of the above (3), wherein the N-acyl derivative of hydroxyproline is an N-acetyl derivative, an N-propionyl derivative, an N-butyryl derivative or an N-isobutyryl derivative.
(5) A food additive or a feed additive for preventing or improving a dry state of the skin, which comprises hydroxyproline, an N-acyl derivative of hydroxyproline or a salt thereof as an active ingredient.
(6) The food additive or the feed additive of the above (5), wherein the N-acyl derivative of hydroxyproline is an N-acetyl derivative, an N-propionyl derivative, an N-butyryl derivative or an N-isobutyryl derivative.
(7) A food and drink comprising hydroxyproline, an N-acyl derivative of or a salt thereof and having an indication of the use for preventing or improving a dry state of the skin.
(8) A food and drink comprising hydroxyproline, an N-acyl derivative of hydroxyproline or a salt thereof and having an indication of a function of preventing or improving a dry state of the skin in the substance, package, manufacture's instruction, advertisement or electronic information for advertisement.
(9) A method for preventing or improving a dry state of the skin, which comprises orally administering hydroxyproline, an N-acyl derivative of hydroxyproline or a salt thereof.
(10) The method of the above (9), wherein the N-acyl derivative of hydroxyproline is an N-acetyl derivative, an N-propionyl derivative, an N-butyryl derivative or an N-isobutyryl derivative.
(11) Use of hydroxyproline, an N-acyl derivative of hydroxyproline or a salt thereof for production of an orally administered agent for preventing or improving a dry state of the skin.
(12) Use of the above (11), wherein the N-acyl derivative of hydroxyproline is an N-acetyl derivative, an N-propionyl derivative, an N-butyryl derivative or an N-isobutyryl derivative.

### Effect of the Invention

The present invention can provide safe orally administered agents, foods and drinks, feeds, food additives or feed additives for preventing or improving a dry state of the skin.

### Brief Description of the Drawing

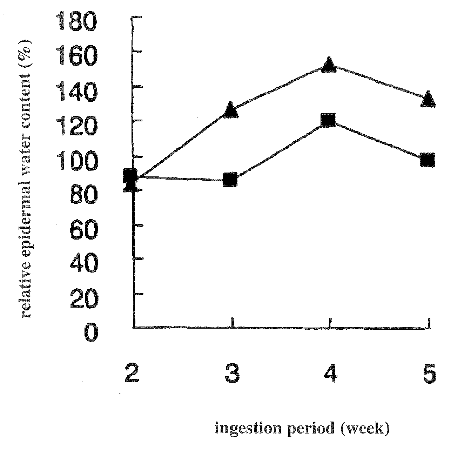

[Fig. 1] Fig. 1 is a graph showing a change in relative epidermal water content with time. A black square indicates a 0.5% hydroxyproline ingestion group (second group), and a black triangle indicates a 1% hydroxyproline ingestion group (third group). The ordinate of the graph represents a relative epidermal water content, and the abscissa of the graph represents an ingestion period of hydroxyproline.

### Best Mode for Carrying Out the Invention

Hydroxyproline used in the present invention be any of stereoisomers of hydroxyproline. There are eight types of stereoisomers depending upon whether proline of hydroxyproline is a D-isomer or an L-isomer, whether the position of the hydroxyl group is a 3-position or a 4-position and whether the stereoisomer is a cis-form or a trans-form, and all of them can be used as stereoisomers.
Specific examples of hydroxyproline include cis-4-hydroxy-L-proline, cis-4-hydroxy-D-proline, cis-3-hydroxyL-proline, cis-3-hydroxy-D-proline, trans-4-hydroxy-L-proline, trans-4-hydroxy-D-proline, trans-3-hydroxy-L-proline and trans-3-hydroxy-D-proline.

Hydroxyproline is one of amino acids which widely exist in nature as major constitutive amino acids of collagen and constitutive amino acids of elastin, and it can be produced by subjecting collagen derived from animals such as a pig and cattle to acid hydrolysis and purifying the hydrolyzate by an ordinary method.
Trans-4-hydroxy-L-proline can be produced using a proline 4-hydroxylase isolated from microorganisms of the genus Amycolatopsis or Dactylosporangium (Japanese Published Unexamined Patent Application No. 313179/95). Cis-3-hydroxy-L-proline can be produced using a proline 3-hydroxylase isolated from microorganisms of the genus Streptomyces (Japanese Published Unexamined Patent Application No. 322885/95) (Bioindustry, vol. 14, No. 31, 1997).

Hydroxyproline produced using the enzymes derived from the foregoing microorganisms is excellent in quality, and preferable as hydroxyproline used in the present invention.
As the N-acyl derivative of hydroxyproline used in the present invention, the foregoing N-acyl derivatives of hydroxyproline stereoisomers can be mentioned. The acyl group of the N-acyl derivative includes an acyl group having 1 to 24 carbon atoms, preferably 1 to 12 carbon atoms, especially preferably 1 to 6 carbon atoms. Specific examples thereof include formyl, acetyl, propionyl, butyryl, isobutyryl, valeryl, pivaloyl, hexanoyl, heptanoyl, octanoyl, nonanoyl, decanoyl, undecanoyl, dodecanoyl and the like. Especially, acetyl, propionyl, butyryl and isobutyryl are preferable.

Examples of the salt of hydroxyproline or the N-acyl derivative of hydroxyproline include alkali metal salts such as sodium, potassium and lithium, alkaline earth metal salts such as calcium and magnesium, ammonium salt, addition salts of amines such as monoethanolamine, diethanolamine, triethanolamine and triisopropanolamine, addition salts of basic amino acids such as arginine and lysine, and the like.

The N-acyl derivative of hydroxyproline can be prepared by a known method. For example, the N-acyl derivative of hydroxyproline can be prepared by converting a linear or branched, saturated or unsaturated fatty acid having 1 to 24 carbon atoms to halide such as chloride or bromide using a halogenating agent such as thionyl chloride or phosgene and then condensing the halide with the foregoing hydroxyproline or converting the fatty acid to an acid anhydride and then reacting the acid anhydride with hydroxyproline.

As the fatty acid, fatty acids such as formic acid, acetic acid, propionic acid, butyric acid, isobutyric acid, valeric acid, isovaleric acid, pivalic acid, hexanoic acid, heptanoic acid, octanoic acid, nonanoic acid, decanoic acid, undecanoic acid and dodecanoic acid are used either solely or in combination.
A process for producing the N-acyl derivative of hydroxyproline via acid halide is described below.

A fatty acid is dispersed in a solvent such as methylene chloride, chloroform, carbon tetrachloride, benzene, toluene, xylene or n-hexane, and 1 to 5-fold equivalents of halogenating agent is added thereto for reaction to obtain a fatty acid halide. Subsequently, hydroxyproline is dissolved or dispersed in the solvent. While the resulting solution is maintained at 5 to 70°C, 0.3 to 3.0-fold equivalents of fatty acid halide is added based on hydroxyproline to conduct an acylation reaction, whereby the N-acyl derivative of hydroxyproline can be prduced.

Examples of the solvent used in the acylation reaction include water, methanol, ethanol, isopropanol, isobutanol, acetone, toluene, tetrahydrofuran, ethyl acetate, N, N-dimethylformamide, dimethyl sulfoxide and the like. These may be used either solely or in combination. When hydroxyproline is dissolved or dispersed in the solvent, 0.8 to 2.0-fold equivalents of alkaline substance such as sodium hydroxide or potassium hydroxide based on hydroxyproline may be dissolved or dispersed in the solvent as required.

To obtain a salt of the N-acyl derivative of hydroxyproline, when the N-acyl derivative of hydroxyproline is obtained in the form of a salt, it may be purified as it is. When it is obtained in a free form, it may be dissolved or suspended in a suitable solvent, followed by addition of a base to form a salt.
In the purification, an ordinary method such as crystallization or chromatography is used.

Specific examples of the N-acyl derivative of hydroxyproline include N-acetyl-cis-4-hydroxy-L-proline, N-acetyl-cis-4-hydroxy-D-proline, N-acetyl-cis-3-hydroxyL-proline, N-acetyl-cis-3-hydroxy-D-proline, N-acetyl-trans-4-hydroxy-L-proline, N-acetyl-trans-4-hydroxy-D-proline, N-acetyl-trans-3-hydroxy-L-proline, N-acetyl-trans-3-hydroxy-D-proline, N-propionyl-cis-4-hydroxy-L-proline, N-propionyl-cis-4-hydroxy-D-proline, N-propionyl-cis-3-hydroxy-L-proline, N-propionyl-cis-3-hydroxy-D-proline, N-propionyltrans-4-hydroxy-L-proline, N-propionyl-trans-4-hydroxy-D-proline, N-propionyl-trans-3-hydroxy-L-proline, N-propionyl-trans-3-hydroxy-D-proline, N-butyryl-cis-4-hydroxy-L-proline, N-butyryl-cis-4-hydroxy-D-proline, N-butyryl-cis-3-hydroxy-L-proline, N-butyryl-cis-3-hydroxy-D-proline, N-butyryl-trans-4-hydroxy-L-proline, N-butyryl-trans-4-hydroxy-D-proline, N-butyryl-trans-3-hydroxy-L-proline, N-butyryl-trans-3-hydroxy-D-proline, N-isobutyryl-cis-4-hydroxy-L-proline, N-isobutyryl-cis-4-hydroxy-D-proline, N-isobutyryl-cis-3-hydroxy-L-proline, N-isobutyryl-cis-3-hydroxy-D-proline, N-isobutyryl-trans-4-hydroxy-L-proline, N-isobutyryl-trans-4-hydroxy-D-proline, N-isobutyryl-trans-3-hydroxy-L-proline, N-isobutyryl-trans-3-hydroxy-D-proline and the like.

In the orally administered agent, the food and drink, the feed, the food additive or the feed additive of the present invention, as hydroxyproline, the N-acyl derivative of hydroxyproline or the salt thereof, cis-4-hydroxy-L-proline, cis-4-hydroxy-D-proline, cis-3-hydroxyL-proline, cis-3-hydroxy-D-proline, trans-4-hydroxy-L-proline, trans-4-hydroxy-D-proline, trans-3-hydroxy-L-proline, trans-3-hydroxy-D-proline, various N-acyl derivatives of these and the salts thereof can be used either solely or in combination.

The orally administered agent, the food and drink, the feed, the food additive or the feed additive of the present invention may contain, in addition to the foregoing essential component, additives suitable for each use as required.
The water content of the skin is increased by administering or ingesting the orally administered agent, the food and drink, the feed, the food additive or the feed additive of the present invention, so that the dry state of the skin can be prevented or improved.

The dry state of the skin includes a dry state of the skin accompanied by atopic dermatitis, xeroderma, chapped hand, chapped skin or the like.
The chapped hand refers to a symptom caused by irritating the hand when a person whose skin is easily dried constitutionally because of less keratin intracellular lipid or the like does scrubbing and washing such as kitchen work or laundering or repeatedly uses chemicals such as a shampoo and a hair dye.
The chapped skin refers to a symptom that the stratum corneum is roughened by drying and a moist feeling is lost when the surface of the skin is touched.

The orally administered agent of the present invention contains hydroxyproline, the N-acyl derivative of hydroxyproline or the salt thereof and may contain one or more pharmaceutically acceptable carriers as required and also active ingredients for another treatment as required.
The orally administered agent of the present invention can be prepared by mixing hydroxyproline, the N-acyl derivative of hydroxyproline or the salt thereof with the carriers as required and then subjecting the mixture to any method well-known in the technical field of pharmaceutics.

When the orally administered agent of the present invention is formulated, additives such as excipients, binders, disintegrators, lubricants, dispersing agents, suspending agents, emulsifiers, diluents, buffers, antioxidants and anti-bacterial agents can be used.
Examples of the dosage form of the orally administered agent include tablets, powders, granules, emulsions, syrups, capsules and the like.
For example, when the dosage form is tablets, powders, granules or the like, the formulation can be conducted by adding saccharides such as lactose, glucose, sucrose, mannitol and sorbitol, starches such as potato starch, wheat starch and corn starch, inorganic substances such as calcium carbonate, calcium sulfate, sodium hydrogencarbonate and sodium chloride, excipients, for example, powdered plants such as powdered glycyrrhiza and powdered gentian, disintegrators such as starch, agar, gelatin powder, crystalline cellulose, carmellose sodium, carmellose calcium, calcium carbonate, sodium hydrogencarbonate and sodium alginate, lubricants such as magnesium stearate, talc, hydrogenated vegetable oil, macrogol and silicone oil, binders such as polyvinyl alcohol, hydroxypropyl cellulose, methyl cellulose, ethyl cellulose, carmellose, gelatin and starch paste solution, surfactants such as fatty acid esters, plasticizers such as glycerin, and the like.

When the dosage form of the orally administered agent is a liquid preparation such as syrup, formulation can be conducted by adding water, saccharides such as sucrose, sorbitol and fructose, glycols such as polyethylene glycol and propylene glycol, oils such as sesame oil, olive oil and soybean oil, antiseptics such as p-hydroxybenzoic acid ester, flavors such as strawberry flavor and peppermint, and the like.

The concentration of hydroxyproline, the N-acyl derivative of hydroxyproline or the salt thereof in the orally administered agent of the present invention is properly selected according to the type of the orally administered agent, the effect to be expected by administration of the orally administered agent and the like. It is usually 0.1 to 90% by weight, preferably 0.5 to 70% by weight, especially preferably 1 to 50% by weight as the N-acyl derivative of hydroxyproline or the salt thereof.

The dose of the orally administered agent of the present invention may vary depending upon the dosage form, and the age weight of a person to which the agent is being administered, and the like. It is usually 100 to 10,000 mg, preferably 100 to 2,000 mg, especially preferably 200 to 1,000 mg a day for an adult patient as hydroxyproline, the N-acyl derivative of hydroxyproline or the salt thereof. The orally administered agent is administered once or in several divided portions a day. Although the administration period is not particularly limited, it is usually one day to one year, preferably one week to three months.

The food additive of the present invention can be prepared in the same manner as the orally administered agent. The food additive is usually prepared by mixing or dissolving other food additives as required and processing the mixture in the form of, for example, powders, granules, pellets, tablets or liquid preparations.
As the food and drink of the present invention, those obtained by adding hydroxyproline, the N-acyl derivative of hydroxyproline or the salt thereof, or the food additive of the invention to food and drink can be mentioned.

The food and drink of the present invention can be prepared by a general method for preparing foods and drinks except that hydroxyproline, the N-acyl derivative of hydroxyproline or the salt thereof, or the food additive of the invention is added to food and drink.
The food and drink of the present invention can be prepared by a granulation method such as fluidized bed granulation, stirring granulation, extrusion granulation, rolling granulation, air stream granulation, compression molding granulation, disruption granulation, spray granulation or blasting granulation, a coating method such as pan coating, fluidized bed coating or dry coating, a plumping method such as puff drying, an excess steam method, a foam mat method or a microwave heating method and an extrusion method with an extrusion granulator or an extruder.

The food and drink of the present invention may be in the form of juices, soft drinks, tea, lactic acid bacteria beverages, fermented milk, frozen dessert, dairy products such as butter, cheese, yogurt, processed milk and skim milk, animal meat products such as ham, sausages and hamburger, fish meat paste products such as *kamaboko* (boiled fish paste), *chikuwa* (stick-shaped fish cake) and *satumaage* (fried fish meat), egg products such as *dashimaki* (omlet with stock) and *tamagodofu* (steamed egg-tofu), confectionery such as cookies, jellies, chewing gum, candies and snacks, bread, noodles, pickles, smoked products, dried fish, foods boiled down in soy sauce, salt curing products, soups, seasonings and the like.

The food and drink of the present invention may be in the form of any of powdered foods, sheet-shaped foods, bottled foods, canned foods, retort foods, capsule foods, tablet-like foods, liquid foods, drinks and the like.
The food and drink of the invention can be used as food and drink such as health foods or functional foods for preventing or improving the dry state of the skin.

The food and drink or the food additive of the invention may contain food additives which are generally used in foods and drinks, such as sweeteners, coloring agents, preservatives, thickening stabilizers, antioxidants, color-developing agents, bleaching agents, fungicides, gum bases, bitter agents, enzymes, wax, sour agents, seasonings, emulsifier, nutrient supplements, additional materials for preparation, flavors and spice extracts.
The amount of hydroxyproline, the N-acyl derivative of hydroxyproline or the salt thereof, or the food additive of the present invention to be added to the food and drink of the present invention is properly selected according to the types of the food and drink, the effect to be expected by ingestion of the food and drink, and the like. It is added in an amount of, usually 0.1 to 90% by weight, preferably 0.5 to 70% by weight, especially preferably 1 to 50% by weight as hydroxyproline, the N-acyl derivative of hydroxyproline or the salt thereof.

The ingestion amount of the food and drink of the present invention varies depending upon the ingestion form, the age and weight of a person who is being ingested to, and the like. It is usually 100 to 10,000 mg, preferably 100 to 2,000 mg, especially preferably 200 to 1,000 mg a day for an adult as hydroxyproline, the N-acyl derivative of hydroxyproline or the salt thereof. The food and drink are ingested once or in several divided portions a day.
The ingestion period is not particularly limited. It is usually one day to one year, preferably one week to three months.

The feed additive of the present invention can be prepared by the same method as the orally administered agent of the present invention. The feed additive is prepared by mixing or dissolving other feed additives as required and formulating the mixture into powders, granules, pellets, tablets, liquid preparations or the like.
The feed of the present invention may be any of a feed for pets, a feed for domestic animals, a feed for poultry and the like so long as it is a feed for preventing or improving a dry state of the skin of animals such as mammals and birds. For example, it can advantageously be used as a feed for preventing or improving a dry state of the skin for pets such as monkeys, dogs, cats, rats and mice.

The feed of the present invention can be prepared by a general method for producing feeds except that hydroxyproline, the N-acyl derivative of hydroxyproline or the salt thereof, or the feed additive of the present invention is added to a feed.
Examples of the feed include grains, bran, vegetable oil cakes, animal feeds, other feeds, purified products, mixtures thereof and the like.

Examples of the grains include milo, wheat, barley, oats, rye, brown rice, buckwheat, foxtail millet, broomcorn millet, Japanese millet, corn, soybean and the like.
Examples of the bran include rice bran, defatted rice bran, wheat bran, wheat middlings, wheat, germ, barley bran, pellets, corn bran, corn germ the like.
Examples of the vegetable oil cakes include soybean oil cake, soybean flour, linseed oil cake, cottonseed oil cake, peanut oil cake, safflower oil cake, coconut oil cake, palm oil cake, sesame oil cake, sunflower oil cake, rape seed oil cake, kapok oil cake, mustard oil cake and the like.

Examples of the animal feeds include fishmeal such as northern ocean meal, imported meal, whole meal and coastal meal, fish soluble, meat meal, meat and bone meal, blood powder, degraded hair, bone meal, treated byproducts for livestock, feather meal, silkworm pupa, skim milk, casein, dry whey and the like.
Examples of other feeds include stalks and leaves of plants such as alfalfa, hay cube, alfalfa leaf meal and false acacia powder, byproducts from the corn processing industry such as corn gluten, meal, corn gluten feed and corn steep liquor, processed starch products such as starch, products from the fermentation industry such as yeast, beer cake, malt root, alcohol cake and soy sauce cake, agricultural byproducts such as processed citrus fruit cake, tofu cake, coffee cake and cocoa cake, cassava, broad bean, guar meal, seaweeds, krill, spirulina, chlorella, minerals and the like.

Examples of the purified products include proteins such as casein and albumin, amino acids, sugars such as starch, cellulose, sucrose and glucose, minerals, vitamins and the like.
The feed of the invention may be prepared by a granulation method such as fluidized bed granulation, stirring granulation, extrusion granulation, rolling granulation, air stream granulation, compression molding granulation, disruption granulation, spray granulation or blasting granulation, a coating method such as pan coating, fluidized bed coating or dry coating, a plumping method such as puff drying, an excess steam method, a foam mat method or a microwave heating method, an extrusion method with an extrusion granulator or an extruder, or the like.

The amount of hydroxyproline, the N-acyl derivative of hydroxyproline or the salt thereof, or the feed additive to be added to the feed in the present invention is properly selected according to the type of the feed, the effect to be expected by ingestion of the feed and the like. It is added in an amount of, usually 0.1 to 90% by weight, preferably 0.5 to 70% by weight, especially preferably 1 to 50% by weight as hydroxyproline, the N-acyl derivative of hydroxyproline or the salt thereof.

The ingestion amount of the feed in the present invention varies depending upon the ingestion form, the type of the ingestion animal, the age and weight of the animal and the like. It is usually 10 to 1,000 mg, preferably 10 to 200 mg, especially preferably 20 to 100 mg per kg of the weight a day as hydroxyproline, the N-acyl derivative of hydroxyproline or the salt thereof. The feed is ingested once or in several divided portions a day. The ingestion period is not particularly limited. It is usually one day to one year, preferably one week to three months.

Test Examples are described below in which an effect of increase in skin water content and an effect of improvement in keratin barrier function by oral ingestion of hydroxyproline were examined.

### Test Example 1

HOS:HR-1 mice (male, 5 weeks old, purchased from SLC Japan) were used in the test. The breeding conditions were that the room temperature was 22 ± 2°C, the humidity was 35 ± 15% and a feed and water were freely ingested.

Each test group consisted of 10 mice. A commercially available powder feed CE-2 (manufactured by Clea Japan) was fed to mice of the first group, CE-2 containing 0.5% by weight of trans-4-hydroxy-L-proline (hereinafter also referred to simply as hydroxyproline; manufactured by Kyowa Hakko Kogyo Co., Ltd.) was fed to mice of the second group, and CE-2 containing 1% by weight of hydroxyproline was fed to mice of the third group.

The water content of the skin surface on the back thigh upper portion of each mouse was measured with SKICON-200 (manufactured by IBS) every one week, from two weeks to five weeks after the start-up of the feeding of each feed. The measurement was conducted ten times for each mouse, and an average value of each mouse was obtained. Further, an average value of each test group was calculated.
The relative epidermal water content was calculated according to the following formula using the average value. Relative epidermal water content (%) = (A2/A1) x 100
A1: Epidermal water content of the first group
A2: Epidermal water content of the second or third group
The results are shown in Fig. 1.

From Fig. 1, it became clear that the water content of the skin is markedly improved by the oral ingestion of hydroxyproline.

### Test Example 2

HOS:HR-1 mice (female, 4 weeks old, purchased from SLC Japan) were used in the test. The breeding conditions were that the room was 22 ± 2°C, the humidity was 50 ± 5% and a feed and water were freely ingested.

Each test group consisted of 10 mice. A commercially available powder feed CE-2 was fed to mice of the first group, a special feed (manufactured by Nippon Nosan Kogyo K.K.) was fed to mice of the second group, the special feed containing 0.5% by weight of hydroxyproline was fed to mice of the third group, and the special feed containing 1% by weight of was fed to mice of the fourth group. The dry skin was induced in HOS:HR-1 mice by feeding of the special feed (The journal of international medical research pharmacology, 32, 392-399 (2004)).

A transepidermal water loss (TEWL) value of the right rump of each mouse was measured as an index of a barrier function of stratum corneum at the start-up of the feeding of each feed, after four weeks and after six weeks using Tewameter TM210 (Courage + Khazaka electronic GmbH, Germany), and an average value of each group was calculated. In a test of a significant difference, a t-test was conducted, and a risk rate of less than 5% was defined as a significant difference.

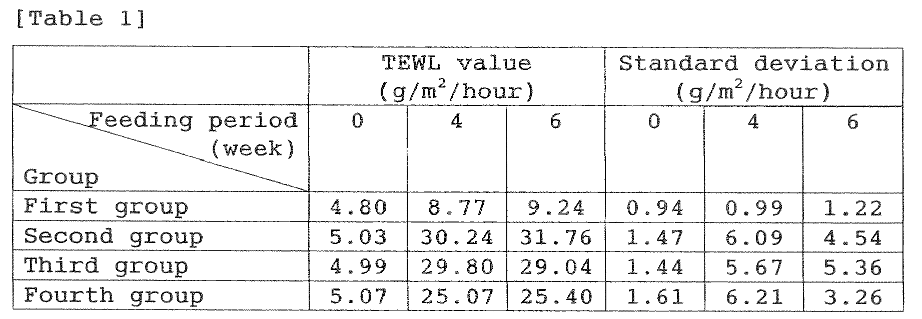

The results are shown in Table 1.
In the second group, the transepidermal water loss was significantly increased in comparison with the first group. Meanwhile, in the third and fourth groups, the increase in transepidermal water loss was suppressed dependently on the concentration of hydroxyproline, and the significant difference was obtained in the fourth group in comparison with the second group.
The foregoing results revealed the effect of improving the barrier function of stratum corneum by the oral ingestion of hydroxyproline.
Examples of the invention are described below.

### Example 1

### Production of tablets containing hydroxyproline

Tablets containing trans-4-hydroxy-L-proline are produced by an ordinary method. That is, the following components are uniformly mixed, and the mixture is tableted with a single punch tablet machine to obtain tablets each having a diameter of 5 mm and a weight of 15 mg.

| Components | Amount |
|---|---|
| trans-4-hydroxy-L-proline | 10.0 g |
| lactose | 90.0 g |
| dry corn starch | 2.0 g |
| talc | 1.8 g |
| magnesium stearate | 0.2 g |

### Example 2

### Production of granules containing hydroxyproline

The tablets obtained in Example 1 are milled, granulated and sieved to obtain granules of 20 to 50 mesh.

### Example 3

### Production of a drink containing hydroxyproline

A drink containing trans-4-hydroxy-L-proline is produced by uniformly stirring and dissolving the following components and adding purified water to adjust the total volume to 1,000 ml. A suitable amount of a flavor or a pigment in the following components refers to an amount which is ordinarily used in the production of drinks, and a suitable amount of purified water refers to an amount required for adjusting the total volume to 1,000 ml by addition to other components.

| Components | Amount |
|---|---|
| trans-4-hydroxy-L-proline | 5.0 g |
| sodium benzoate | 1.0 g |
| fructose | 10.0 g |
| flavor | suitable amount |
| pigment | suitable amount |
| purified water | suitable amount |

### Example 4

### Production of candies containing N-acetylhydroxyproline

N-acetyl-trans-4-hydroxy-L-proline-containing candies comprising the following components are produced by an ordinary method.

| Components | Amount |
|---|---|
| N-acetyl-trans-4-hydroxy-L-proline | 1.00 g |
| sorbitol powder | 98.75 g |
| flavor | 0.20 g |
| sorbitol seed | 0.05 g |

### Example 5

### Production of a feed containing hydroxyproline

A trans-4-hydroxy-L-proline-containing animal feed comprising the following components is produced by an ordinary method.

| Components | Amount |
|---|---|
| trans-4-hydroxy-L-proline | 1.0 g |
| lard | 5.0 g |
| corn oil | 1.0 g |
| sucrose | 20.0 g |
| cellulose | 5.0 g |
| choline chloride | 0.2 g |
| vitamin mixture | 1.0 g |
| mineral mixture | 3.5 g |
| corn starch | 44.3 g |

### Industrial Applicability

The present invention can provide safe orally administered agents, foods and drinks, feeds, food additives or feed additives for preventing or improving a dry state of the skin.

## Claims

1. An orally administered agent for preventing or improving a dry state of the skin, which comprises hydroxyproline, an N-acyl derivative of hydroxyproline or a salt thereof as an active ingredient.

2. The orally administered agent according to claim 1, wherein the N-acyl derivative of hydroxyproline is an N-acetyl derivative, an N-propionyl derivative, an N-butyryl derivative or an N-isobutyryl derivative.

3. A food and drink or a feed for preventing or improving a dry state of the skin, which comprises hydroxyproline, an N-acyl derivative of hydroxyproline or a salt thereof as an active ingredient.

4. The food and drink or the feed according to claim 3, wherein the N-acyl derivative of hydroxyproline is an N-acetyl derivative, an N-propionyl derivative, an N-butyryl derivative or an N-isobutyryl derivative.

5. A food additive or a feed additive for preventing or improving a dry state of the skin, which comprises hydroxyproline, an N-acyl derivative of hydroxyproline or a salt thereof as an active ingredient.

6. The food additive or the feed additive according to claim 5, wherein the N-acyl derivative of hydroxyproline is an N-acetyl derivative, an N-propionyl derivative, an N-butyryl derivative or an N-isobutyryl derivative.

7. A food and drink comprising hydroxyproline, an N-acyl derivative of hydroxyproline or a salt thereof and having an indication of the use for preventing or improving a dry state of the skin.

8. A food and drink comprising hydroxyproline, an N-acyl derivative of hydroxyproline or a salt thereof and having an indication of a function of preventing or improving a dry state of the skin in the substance, package, manufacture's instruction, advertisement or electronic information for advertisement.

9. A method for preventing or improving a dry state of the skin, which comprises orally administering hydroxyproline, an N-acyl derivative of hydroxyproline or a salt thereof.

10. The method according to claim 9, wherein the N-acyl derivative of hydroxyproline is an N-acetyl derivative, an N-propionyl derivative, an N-butyryl derivative or an N-isobutyryl derivative.

11. Use of hydroxyproline, an N-acyl derivative of hydroxyproline or a salt thereof for production of an orally administered agent for preventing or improving a dry state of the skin.

12. Use according to claim 11, wherein the N-acyl derivative of hydroxyproline is an N-acetyl derivative, an N-propionyl derivative, an N-butyryl derivative or an N-isobutyryl derivative.
